# EUROPÄISCHE PATENTANMELDUNG

(11) **EP 3 312 154 A1**
(43) Veröffentlichungstag der Anmeldung: **25.04.2018**
(21) Anmeldenummer: 16194960.7
(22) Anmeldetag: 21.10.2016
(51) Int. Cl.: C07C 2/84, C07C 11/04, B01J 19/00

(54) **VERFAHREN ZUR GEWINNUNG VON ETHYLEN AUS METHAN**

(71) Anmelder: Linde Aktiengesellschaft, 80331 München (DE)
(72) Erfinder: OBERLE, David, 81476 München (DE)
(74) Vertreter: Frommberger, Moritz

(57) **Zusammenfassung**

Verfahren zur Gewinnung von Ethylen aus Methan, bei dem ein oder mehrere Methan enthaltende Einsatzgasgemische vorgewärmt und einem oder mehreren Reaktoren (1) zugeführt werden, in dem oder denen ein Teil des in dem oder den Einsatzgasgemischen enthaltenen Methans durch oxidative Kopplung zu Ethylen umgesetzt wird, wobei das Vorwärmen des oder der Einsatzgemische unter Verwendung eines oder mehrere Wärmetauscher (2) durchgeführt wird, in dem oder denen ein oder mehrere Produktgemische, das oder die dem oder den Reaktoren (1) entnommen werden, abgekühlt werden, und wobei das Vorwärmen des oder der Einsatzgemische während eines Anfahrzeitraums zusätzlich unter Verwendung eines mit Brennstoff befeuerten Gaserhitzers (3) durchgeführt wird, dadurch gekennzeichnet, dass das oder die Einsatzgasgemische zumindest während des Anfahrzeitraums in paralleler Stromführung durch den oder die Wärmetauscher (2) und durch den Gaserhitzer (3) geführt werden. Eine entsprechende Anlage (100) ist ebenfalls Gegenstand der Erfindung.

## Beschreibung

Die Erfindung betrifft ein Verfahren zur Gewinnung von Ethylen aus Methan und eine entsprechende Anlage gemäß den Oberbegriffen der unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet.

Die Reaktionsbedingungen bei der oxidativen Kopplung von Methan umfassen klassischerweise eine Temperatur von 500 bis 900 °C und einen Druck von 1 bis 10 bar sowie hohe Raumgeschwindigkeiten. Jüngere Entwicklungen gehen insbesondere auch in Richtung der Verwendung tieferer Temperaturen. Im vorliegenden Fall wird von einer Temperatur von ca. 520 °C ausgegangen. Die Re aktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden; der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Typischerweise werden nicht mehr als 10 bis 15% des eingesetzten Methans umgesetzt. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen überwiegend nicht umgesetztes Methan sowie Kohlendioxid, Kohlenmonoxid und Wasser. Durch ggf. erfolgende nichtkatalytische Spaltreaktionen können auch beträchtliche Mengen an Wasserstoff enthalten sein. Ein derartiges Gasgemisch wird im hier verwendeten Sprachgebrauch auch als "Produktgemisch" der oxidativen Kopplung von Methan bezeichnet, obwohl es überwiegend nicht die gewünschten Produkte, sondern auch das nicht umgesetzte Edukt Methan und die soeben erläuterten Nebenprodukte enthält.

Bei der oxidativen Kopplung von Methan können Reaktoren eingesetzt werden, in denen einer katalytischen Zone eine nichtkatalytische Zone nachgeschaltet ist. Das aus der katalytischen Zone ausströmende Gasgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann.

Ein derartiges gezieltes Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet. Auch derartige Ausgestaltungen sind von der vorliegenden Erfindung umfasst, wie unten erläutert.

Bevor einem Reaktor zur oxidativen Kopplung von Methan ein Einsatzgasgemisch, das insbesondere Methan und ggf. auch Sauerstoff enthält, zugeführt wird, wird dieses auf die erforderlichen Reaktionstemperaturen von, wie erwähnt, beispielsweise ca. 520 °C vorgewärmt. Dies erfolgt typischerweise unter Verwendung eines oder mehrerer Abwärmetauscher, die zum Abkühlen des Produktgemischs verwendet werden.

Steht in einem Anfahrbetrieb einer entsprechenden Anlage bzw. in einem Anfahrzeitraum in einem entsprechenden Verfahren keine ausreichende Abwärmemenge zur Verfügung, muss das Einsatzgasgemisch unter Verwendung zusätzlicher Mittel erwärmt werden. Hierzu können unter Verwendung eines Brennstoffs befeuerte Gaserhitzer eingesetzt werden. Bekannte Lösungen besitzen jedoch beträchtliche Nachteile, die auch nachfolgend noch erläutert werden.

Die vorliegende Erfindung stellt sich die Aufgabe, derartige Nachteile bekannter Verfahren und Vorrichtungen zur Gewinnung von Ethylen aus Methan zu beheben.

### Offenbarung der Erfindung

Vor diesem Hintergrund schlägt die vorliegende Erfindung ein Verfahren und eine Anlage zur Gewinnung von Ethylen aus Methan mit den Merkmalen der unabhängigen Patentansprüche vor. Ausgestaltungen sind jeweils Gegenstand der abhängigen Patentansprüche sowie der nachfolgenden Beschreibung.

### Vorteile der Erfindung

Die vorliegende Erfindung geht von einem Verfahren zur Gewinnung von Ethylen aus Methan aus, bei dem ein oder mehrere Methan enthaltende Einsatzgasgemische vorgewärmt und einem oder mehreren Reaktoren zugeführt werden, in dem oder denen ein Teil des in dem oder den Einsatzgasgemischen enthaltenen Methans durch oxidative Kopplung zu Ethylen umgesetzt wird. Wie auch nachfolgend noch im Detail erläutert, kann die vorliegende Erfindung insbesondere auch für parallelisierte Reaktorkonzepte eingesetzt werden, also Verfahren bzw. Anlagen, in denen parallel mehrere Reaktoren vorgesehen sind und jeweils mit einem Einsatzgasgemisch beschickt werden. Die parallel angeordneten Reaktoren können dabei mit Einsatzgasgemischen identischer Zusammensetzung beschickt werden, die beispielsweise von einer gemeinsamen Hauptleitung ausgehend auf die einzelnen Reaktoren verteilt werden. In entsprechender Weise werden den einzelnen Reaktoren auch jeweils Produktgemische entnommen, die, bis auf reaktorindividuelle Schwankungen, ebenfalls im Wesentlichen die gleiche Zusammensetzung aufweisen können. Diese Produktgemische können in einer gemeinsamen nachgeordneten Einrichtung aufbereitet werden.

Wie erwähnt, erstreckt sich die vorliegende Erfindung auch auf Verfahren und Anlagen, bei denen ein postkatalytisches Dampfspalten durchgeführt wird. In Reaktoren, die in entsprechenden Verfahren und Anlagen eingesetzt werden, sind jedoch immer Reaktoren vorhanden, in denen ein Teil des in dem oder den Einsatzgasgemischen enthaltenen Methans durch oxidative Kopplung zu Ethylen umgesetzt wird, und denen Produktgemische entnommen werden.

Im Rahmen der vorliegenden Erfindung ist vorgesehen, dass das Vorwärmen des oder der Einsatzgemische unter Verwendung eines oder mehrerer Wärmetauscher durchgeführt wird, in dem oder denen ein oder mehrere Produktgemische, das oder die dem oder den Reaktoren entnommen werden, abgekühlt werden, und dass das Vorwärmen des oder der Einsatzgemische zumindest während eines Anfahrzeitraums zusätzlich unter Verwendung eines mit einem Brennstoff wie einem Brennstoff oder einem flüssigen Brennstoff wie Heizöl befeuerten Gaserhitzers in paralleler Stromführung zu dem oder den Wärmetauschern durchgeführt wird.

Grundsätzlich ist eine Verwendung eines mit Brennstoff befeuerten Gaserhitzers während eines Anfahrzeitraums bekannt. Allerdings werden hierbei für Verfahren zur oxidativen Kopplung von Methan typischerweise Gaserhitzer eingesetzt, die seriell zu dem oder den Wärmetauschern angeordnet sind, in dem oder denen die weitere Erwärmung im Anfahrbetrieb bzw. die ausschließliche Erwärmung in einem sich anschließenden Produktionsbetrieb vorgenommen wird. Hierbei handelt es sich um den oder die zuvor erwähnten Wärmetauscher, in denen das oder die Produktgemische, das oder die dem oder den Reaktoren entnommen werden, abgekühlt werden. Durch die serielle Anordnung ist ein entsprechender Gaserhitzer stets in den Strom der Einsatzgasgemische eingebunden, und zwar auch dann, wenn dieser nicht mehr benötigt wird, d.h. im regulären Produktionsbetrieb.

Die vorliegende Erfindung schlägt vor, dass das oder die Einsatzgemische während des Anfahrzeitraums in paralleler Stromführung durch den oder die Wärmetauscher und den Gaserhitzer geführt werden. Unter einer "parallelen Stromführung" sei dabei verstanden, dass ein erster Anteil des oder der Einsatzgemische durch den oder die Wärmetauscher und nicht durch den Gaserhitzer und ein zweiter Anteil des oder der Einsatzgemische durch den Gaserhitzer und nicht durch den oder die Wärmetauscher geführt wird.

Der durch einen entsprechenden Gaserhitzer erzeugte Druckverlust wird bei paralleler Stromführung deutlich verringert, weil das oder die Einsatzgemische nur zum Teil durch den Gaserhitzer geführt werden. Bei einer seriellen Anordnung addieren sich hingegen die Druckverluste im Gaserhitzer und dem jeweiligen Wärmetauscher. Ein entsprechender Druckverlust ist insbesondere deshalb von Nachteil, weil ein wesentlicher Anteil des Methans in dem oder den Einsatzgasgemischen rückgeführtes, in dem oder den Reaktoren nicht umgesetztes Methan ist. Dieses wird aus dem oder den Produktgemischen abgetrennt. Jeder Druckverlust muss durch einen höheren Druck in einem entsprechenden Abtrennprozess ausgeglichen werden, was im Falle eines dauerhaft seriell eingebundenen Gaserhitzers zu einem beträchtlichen apparativen wie auch energetischen Mehraufwand führt.

Gemäß einer Ausführungsform der vorliegenden Erfindung werden das oder die Einsatzgasgemische während eines dem Anfahrzeitraum nachfolgenden Produktionszeitraums nicht durch den Gaserhitzer geführt. Hierbei kann im Produktionsbetrieb der Gaserhitzer stillgelegt werden. Dies ist zwar grundsätzlich auch in einer seriellen Anordnung möglich, allerdings bleibt der Gaserhitzer hierbei in den Strom des oder der Einsatzgemische eingebunden und verursacht ggf. die erwähnten höheren Druckverluste.

Aufgrund der parallelen Anordnung von Gaserhitzer und Wärmetauscher(n) reduziert sich im Rahmen der vorliegenden Erfindung ferner die volumetrische Belastung im Gaserhitzer beträchtlich (unter Umständen um die Hälfte), weil dieser nur von einem Teil des oder der Einsatzgasgemische durchströmt werden muss. Auf diese Weise kann ein entsprechender Gaserhitzer einfacher und kostengünstiger ausgeführt werden. Durch die parallele Verschaltung kann ferner die Rauchgastemperatur eines entsprechenden Gaserhitzers ohne weitere aufwendige Maßnahmen auf einen technisch sinnvollen Wert von 150 bis 200 °C reduzi ert werden. Bei einer herkömmlichen seriellen Anordnung muss hingegen eine überhöhte Rauchgastemperatur von typischerweise mehr als 400 °C in Kauf genommen werden.

Gemäß einer vorteilhaften Ausgestaltung der vorliegenden Erfindung wird also der Gaserhitzer während des Produktionszeitraums nicht mit dem Brennstoff befeuert und während des Anfahrzeitraums werden Anteile des oder der Einsatzgasgemische parallel durch den oder die Wärmetauscher und den Gaserhitzer geführt.

In Abhängigkeit von der erreichbaren Methantemperatur am Austritt des Gaserhitzers wird während des Anfahrzeitraums ein gewisser Anteil der Einsatzgasgemische, insbesondere ein Viertel oder mehr, durch den Gaserhitzer und ein weiterer Anteil oder der Rest des oder der Einsatzgasgemische durch den oder die Wärmetauscher geführt. Beispielsweise müssen während des Anfahrzeitraums nur 45 bis 55%, insbesondere 50% des oder der Einsatzgasgemische durch den Gaserhitzer geführt werden.

Wie erwähnt, kann die vorliegende Erfindung insbesondere auch bei parallelisierten Reaktorkonzepten zum Einsatz kommen. Bei diesen werden eine Anzahl von Reaktoren und eine der Anzahl von Reaktoren entsprechende Anzahl von Wärmetauschern verwendet, wobei jedem der Reaktoren ein Einsatzgasgemisch zugeführt wird, das während des Anfahrzeitraums teilweise und während des Produktionszeitraums zumindest teilweise durch jeweils einen der Wärmetauscher geführt wird. Jedem der beispielsweise fünf Reaktoren ist damit also ein Wärmetauscher zugeordnet, durch den jeweils das entsprechende Einsatzgasgemisch vorgewärmt und das entsprechende Produktgemisch abgekühlt wird.

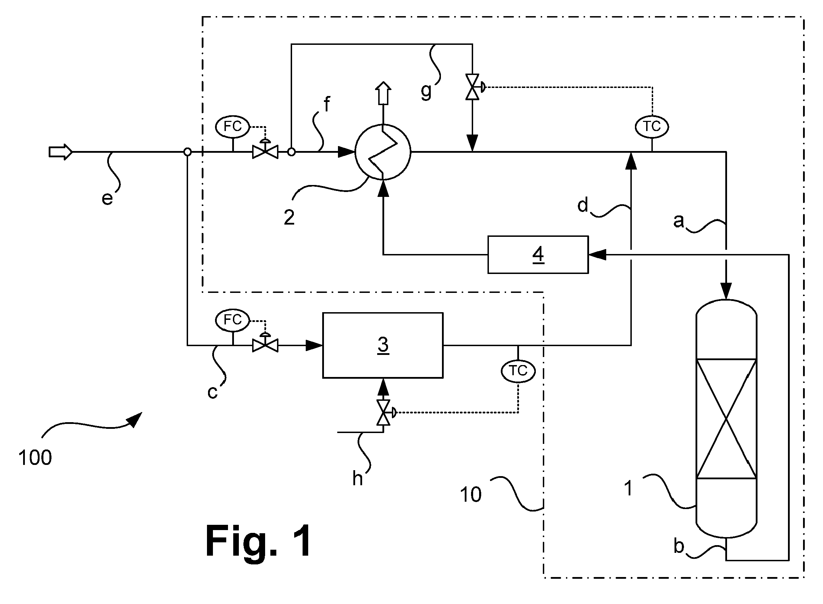

Zu den mehreren Wärmetauschern ist dabei vorteilhafterweise der Gaserhitzer parallel geschaltet. Auf diese Weise kann vorteilhafterweise während des Anfahrzeitraums ein Anteil eines jeden der Einsatzgemische, die den Reaktoren zugeführt werden, parallel in dem Gaserhitzer vorgewärmt werden. Durch die Vorwärmung eines Teils in dem jeweiligen Wärmetauscher und eines weiteren Teils in dem Gaserhitzer können auch unterschiedliche Temperaturen erhalten werden, die zusammen eine Mischtemperatur ergeben. Im Rahmen der vorliegenden Erfindung können dabei zur Temperatursteuerung oder Temperaturregelung Steuerungseinrichtungen und Regler zum Einsatz kommen, wie sie in einem Beispiel unter Bezugnahme auf Figur 1 noch näher erläutert sind.

Ist hier von einem "Anfahrzeitraum" die Rede, handelt es sich hier insbesondere um einen Zeitraum, in dem der oder die Reaktoren noch nicht vollständig hochgefahren sind und sich Menge und/oder Temperatur des oder der Produktgemische insbesondere noch nicht auf einem für einen sich anschließenden Produktionsbetrieb typischem Wert befinden. Das Anfahren mehrerer Reaktoren zur oxidativen Kopplung von Methan erfolgt insbesondere zeitlich versetzt (sequentiell). Der Anfahrbetrieb ist daher beendet, wenn sämtliche Reaktoren hochgefahren sind. Daher umfasst ein Verfahren gemäß einer bevorzugten Ausführungsform der Erfindung, als Anfahrzeitraum einen Zeitraum vorzusehen, in dem eine über das oder die Produktgemische bereitgestellte Wärmemenge unterhalb eines Schwellwerts liegt. Ein Anfahrzeitraum kann jedoch auch beispielsweise als fester Zeitraum ab Inbetriebnahme, Wartung oder Regeneration einer entsprechenden Anlage definiert sein. Ein "Produktionszeitraum" schließt sich dem Anfahrzeitraum an und zeichnet sich insbesondere durch typische Temperaturen, Zusammensetzungen und Mengen des oder der Produktgemische aus.

Im Rahmen der vorliegenden Erfindung kann das Vorwärmen des oder der Einsatzgemische vorteilhafterweise auf ein Temperaturniveau von 450 bis 550 °C, beispielsweise ca. 520 °C, und ausgehend von einem Temperaturniveau von 100 bis 250 °C, beispielsweise ca. 170 °C, durchgeführt. In sbesondere für derartige Temperaturniveaus eignet sich die parallele Verschaltung, wie sie erfindungsgemäß vorgeschlagen wird.

Wie ebenfalls erwähnt, wird im Rahmen der vorliegenden Erfindung das in dem oder den Einsatzgasgemischen enthaltene Methan teilweise aus dem oder den Produktgemischen abgetrennt. Auch hierfür eignet sich die parallele Verschaltung, wie sie erfindungsgemäß vorgeschlagen wird, in besonderer Weise, denn es kann, wie erwähnt, der im Methanrückführweg kritische Druckverlust minimiert werden.

Die Kühlung des oder der Produktgemische muss nicht ausschließlich unter Verwendung des oder der Wärmetauscher durchgeführt werden. Es kann vielmehr auch vorgesehen sein, dass das oder die Produktgemische vor der zumindest teilweisen Abkühlung in dem oder den Wärmetauschern vorgekühlt werden.
Die Vorkühlung des oder der Produktgemische wird dabei auf ein Temperaturniveau von 400 bis 450 °C durchgeführt, beispielsweise auf ca. 427 °C.

Eine Anlage zur Gewinnung von Ethylen aus Methan, mit Mitteln, die dazu eingerichtet sind, ein oder mehrere Methan enthaltende Einsatzgasgemische vorzuwärmen und einem oder mehreren Reaktoren, die dafür eingerichtet sind, einen Teil des in dem oder den Einsatzgasgemischen enthaltenen Methans durch oxidative Kopplung zu Ethylen umzusetzen, zuzuführen, ist ebenfalls Gegenstand der vorliegenden Erfindung. Hierbei sind zum Vorwärmen des oder der Einsatzgemische ein oder mehrere Wärmetauscher bereitgestellt, die dafür eingerichtet sind, ein oder mehrere Produktgemische, das oder die dem oder den Reaktoren entnommen werden, abzukühlen. Ferner ist zum Vorwärmen des oder der Einsatzgemische während eines Anfahrzeitraums zusätzlich ein mit Brennstoff befeuerter Gaserhitzers bereitgestellt.

Erfindungsgemäß sind Mittel vorgesehen, die dafür eingerichtet sind, das oder die Einsatzgasgemische das oder die Einsatzgemische zumindest während des Anfahrzeitraums in paralleler Stromführung durch den oder die Wärmetauscher und den Gaserhitzer zu führen. Insbesondere kann hierbei ein Durchflussregelventil vorgesehen sein, der eine Verteilung des oder der Einsatzgasgemische auf den oder die Wärmetauscher einerseits und den Gaserhitzer andererseits vornimmt.

Eine entsprechende Anlage ist insbesondere zur Durchführung eines Verfahrens eingerichtet, wie es zuvor erläutert wurde, und weist hierzu die entsprechenden Mittel auf. Auf die oben erläuterten Merkmale und Vorteile sei daher an dieser Stelle ausdrücklich verwiesen.

Die Erfindung wird nachfolgend unten mit Bezugnahme auf die beigefügte Zeichnung weiter erläutert, die eine Ausführungsform der Erfindung veranschaulicht.

### Kurze Beschreibung der Zeichnung

Figur 1 veranschaulicht eine Anlage gemäß einer Ausführungsform der Erfindung.

### Ausführliche Beschreibung der Zeichnung

In Figur 1 ist eine Anlage gemäß einer Ausführungsform der Erfindung schematisch veranschaulicht und insgesamt mit 100 bezeichnet. Unter Verwendung der Anlage 100 und der nachfolgend erläuterten Anlagenkomponenten wird zugleich ein Verfahren gemäß einer Ausführungsform der Erfindung durchgeführt, so dass die nachfolgenden Erläuterungen zugleich ein entsprechendes Verfahren betreffen.

Sämtliche strichpunktiert umfasste und insgesamt mit 10 bezeichnete Komponenten sind in der dargestellten Ausführungsform der Erfindung mehrfach vorhanden und parallel angeordnet. Beispielsweise können entsprechende Komponenten jeweils fünffach vorhanden sein. Die vorliegende Erfindung kann sich jedoch, wie zuvor erläutert, auch auf Ausführungsformen erstrecken, in denen entsprechende Komponenten nur in Einzahl vorhanden sind.

Zentrale Komponenten der Anlage 100 sind Reaktoren 1, die für eine oxidative Kopplung von Methan eingerichtet und mit einem geeigneten Katalysator ausgestattet sind. Zu Details sei auf die eingangs zitierte Fachliteratur verwiesen.

Den Reaktoren 1 sind jeweils Wärmetauscher 2 vorgeschaltet, die zum Vorwärmen von den Reaktoren 1 über Leitungen a zugeführten Einsatzgasgemischen verwendet werden. Die Wärmetauscher 2 werden zugleich zum Abkühlen von Produktgemischen verwendet, die den Reaktoren über Leitungen b entnommen werden. Zusätzlich ist zur Erwärmung der Einsatzgemische in einem Anfahrbetrieb der Anlage 100, in der noch keine ausreichende Abwärmemenge zur Verfügung steht, ein befeuerter Gaserhitzer 3 vorgesehen, der allerdings in der hier gezeigten Ausführungsform der Erfindung nur in Einzahl vorhanden und parallel zu den Wärmetauschern 2 angeordnet ist.

Gemäß der hier veranschaulichten Ausführungsform der Erfindung werden also fünf Reaktoren 1 und fünf Wärmetauscher 2 verwendet, wobei jedem der Reaktoren 1 ein Einsatzgasgemisch über eine Leitung a zugeführt wird. Das Anfahren der Reaktoren 1 erfolgt insbesondere zeitlich versetzt (sequentiell). Während des Anfahrbetriebs wird daher insbesondere mit einem entsprechenden zeitlichen Versatz ein Anteil jedes der Einsatzgemische, die den fünf Reaktoren 1 zugeführt werden, in dem Gaserhitzer 3 erwärmt. Im dargestellten Beispiel mit fünf Reaktoren 1 wird bei sequentiellem Anfahren nur ein Fünftel der Leistung benötigt, die bei einem zeitgleichem Anfahren der fünf Reaktoren notwendig wäre. Die Leistung des Gaserhitzers ist also in diesem Fall umgekehrt proportional zur Anzahl der parallelen Reaktoren bzw. Wärmetauscher. Der Gaserhitzer 3 kann entsprechend klein und kostengünstig ausgeführt werden. Eine weitere Erwärmung eines Anteils jedes der Einsatzgemische erfolgt auch während des Anfahrbetriebs in jeweils einem der Wärmetauscher 2, wohingegen in einem sich anschließenden regulären Produktionsbetrieb nur die Wärmetauscher 2 zur Vorwärmung verwendet werden können. Die hier vorzuwärmenden Anteile werden dem Gaserhitzer 3 im Anfahrbetrieb flussratengesteuert oder flussratengeregelt über eine gemeinsame Leitung c zugeführt und anschließend, insbesondere gemäß dem erläuterten sequentiellen Anfahrschema auf mehrere, im dargestellten Beispiel fünf, Leitungen d aufgeteilt.

In einem Zeitraum nach dem Anfahrbetrieb, also in einem regulären Produktionsbetrieb der Anlage 100 ist der Gaserhitzer 3 typischerweise nicht mehr in Betrieb, weil dann die Abwärme auf ausreichend hohem Temperaturniveau in den Wärmetauschern 2 zur Verfügung steht. Die Erwärmung der Einsatzgasgemische erfolgt während des zweiten Zeitraums also typischerweise nur noch über die Wärmetauscher 2.

Zur Bereitstellung der Einsatzgasgemische ist im dargestellten Beispiel eine Hauptleitung e vorgesehen, die beispielsweise einen Recyclestrom aus einem stromabwärtigen Teil der Anlage 100 führen kann. Zu diesem Recyclestrom, der damit Methan enthält, das aus den Produktgemischen abgetrennt wird, wird typischerweise frisches Methan zugespeist. In dem oder den Reaktoren 1 erfolgt eine Zuspeisung von Sauerstoff oder eines sauerstoffhaltigen Gasgemischs.

Die Zuführung in die Wärmetauscher 2 erfolgt, wie die Zuführung in den Gaserhitzer 3, flussratengesteuert oder flussratengeregelt, wobei insbesondere in dem regulären Produktionsbetrieb, eine Flussrate durch den Gaserhitzer 3 auf Null reduziert wird. Die individuelle Zuführung zu den Wärmetauschern 2 erfolgt über Leitungen f. Die Wärmetauscher 2 können insbesondere auch temperaturgesteuert oder temperaturgeregelt über eine Bypassleitung g umgangen werden. Auf diese Weise kann eine Temperatur der Einsatzgasgemische in den Leitungen a eingestellt werden. Eine weitere Einstellmöglichkeit liegt während des Anfahrbetriebs in einer temperaturgesteuerten oder temperaturgeregelten Zufuhr eines Brennstoffs über eine Brennstoffleitung h in den Gaserhitzer 3.

Die Produktgemische, die den Reaktoren über die Leitungen b entnommen werden, liegen zumindest im regulären Produktionsbetrieb auf einem Temperaturniveau von 800°C bis 900°C, beispielsweise ca. 830 °C, und ein em Druckniveau von beispielsweise ca. 8 bar vor. Während der Anfahrphase können in Kühleinrichtungen 4 mit einer Leistung von beispielsweise ca. 27 MW diese Produktgemische auf ein Temperaturniveau von beispielsweise ca. 427 °C abge kühlt werden. Die Abkühlung in den Wärmetauschern 2 kann auf ein Temperaturniveau von beispielsweise ca. 307 °C erfolgen.

Über die Hauptleitung e kann ein Gasgemisch auf einem Temperaturniveau von beispielsweise ca. 170 °C und einem Druckniveau von beispielsweise ca. 10,4 bar bereitgestellt werden. Im Anfahrbetrieb können hiervon beispielsweise ca. 48% durch den Gaserhitzer 3 geführt werden, der Rest durch die Wärmetauscher 2 bzw. die Bypassleitungen g, die diese umgehen. Während des Anfahrbetriebs kann der Gaserhitzer 3 dabei mit einer Heizleistung von beispielsweise ca. 58 MW betrieben werden. Wie erwähnt, ist während des regulären Produktionsbetriebs der Anlage 100 der Gaserhitzer 3 typischerweise nicht in Betrieb.

Während des Anfahrbetriebs wird der Anteil der jeweiligen Einsatzgasgemische in dem Gaserhitzer 3 auf ein Temperaturniveau von beispielsweise ca. 630 °C erwärmt. Auf diese Weise kann eine verminderte Heizleistung in den Wärmetauschern 2 ausgeglichen werden, die nur eine Erwärmung auf beispielsweise ca. 426 °C bewirken. Die Mischtemperatur der Einsatzgasgemische beträgt beispielsweise ca. 528 °C. Durch Druckverluste bedingt werden diese auf einem Druckniveau von beispielsweise ca. 8 bar in die Reaktoren 1 eingespeist.

Ein Druckverlust an den den Wärmetauschern 2 vorgeschalteten Ventilen kann jeweils beispielsweise ca. 2,2 bar im Anfahrbetrieb und ca. 1,3 bar im Produktionsbetrieb betragen.In den Wärmetauschern 2 selbst beträgt der Druckverlust im Anfahrbetrieb beispielsweise nur ca. 0,2 bar während im Produktionsbetrieb bei maximalem Durhchfluss typischerweise 1,1 bar entstehen. In dem dem Gaserhitzer 3 vorgeschalteten Ventil beträgt ein Druckverlust beispielsweise ca. 0,6 bar, in dem Gaserhitzer selbst beispielsweise ca. 1,8 bar. Bei einer seriellen Anordnung von Gaserhitzer 2 und Wärmetauscher(n) 2, wie sie aus dem Stand der Technik bekannt ist, liegt daher ein bis zu doppelt so hoher Gesamtdruckverlust vor als in der in Figur 1 gezeigten parallelen Anordnung, welcher 2,4 bar nicht übersteigt.

## Patentansprüche

1. Verfahren zur Gewinnung von Ethylen aus Methan, bei dem ein oder mehrere Methan enthaltende Einsatzgasgemische vorgewärmt und einem oder mehreren Reaktoren (1) zugeführt werden, in dem oder denen ein Teil des in dem oder den Einsatzgasgemischen enthaltenen Methans durch oxidative Kopplung zu Ethylen umgesetzt wird, wobei das Vorwärmen des oder der Einsatzgemische unter Verwendung eines oder mehrerer Wärmetauscher (2) durchgeführt wird, in dem oder denen ein oder mehrere Produktgemische, das oder die dem oder den Reaktoren (1) entnommen werden, abgekühlt werden, und wobei das Vorwärmen des oder der Einsatzgemische während eines Anfahrzeitraums zusätzlich unter Verwendung eines mit Brennstoff befeuerten Gaserhitzers (3) durchgeführt wird, **dadurch gekennzeichnet, dass** das oder die Einsatzgemische zumindest während des Anfahrzeitraums in paralleler Stromführung durch den oder die Wärmetauscher (2) und den Gaserhitzer (3) geführt werden.

2. Verfahren nach Anspruch 1, bei dem das oder die Einsatzgasgemische während eines dem Anfahrzeitraum nachfolgenden Produktionszeitraums nicht durch den Gaserhitzer (3) geführt werden.

3. Verfahren nach Anspruch 2, bei dem der Gaserhitzer (3) während des Produktionszeitraums nicht mit dem Brennstoff befeuert wird.

4. Verfahren nach einem der vorstehenden Ansprüche, bei dem während des Anfahrzeitraums mehr als ein Viertel des oder der Einsatzgasgemische durch den Gaserhitzer (3) geführt wird.

5. Verfahren nach einem der vorstehenden Ansprüche, bei dem eine Anzahl von Reaktoren (1) und eine der Anzahl von Reaktoren entsprechende Anzahl von Wärmetauschern (2) verwendet werden, wobei jedem der Reaktoren (1) ein Einsatzgasgemisch zugeführt wird, das während des Anfahrzeitraums teilweise und während des Produktionszeitraums zumindest teilweise durch jeweils einen der Wärmetauscher (2) geführt wird.

6. Verfahren nach Anspruch 5, bei dem während des Anfahrzeitraums ein Anteil eines jeden der Einsatzgemische, die den Reaktoren (1) zugeführt werden, in dem Gaserhitzer (3) vorgewärmt wird.

7. Verfahren nach einem der vorstehenden Ansprüche, bei dem der Anfahrzeitraum einem Zeitraum entspricht, in dem eine über das oder die Produktgemische bereitgestellte Wärmemenge unterhalb eines Schwellwerts liegt.

8. Verfahren nach einem der vorstehenden Ansprüche, bei dem das Vorwärmen des oder der Einsatzgemische auf ein Temperaturniveau von 450 bis 550 °C durchgeführt wird.

9. Verfahren nach einem der vorstehenden Ansprüche, bei dem das Vorwärmen des oder der Einsatzgemische ausgehend von einem Temperaturniveau von 100 bis 250 °C durchgeführt wird.

10. Verfahren nach einem der vorstehenden Ansprüche, bei dem das in dem oder den Einsatzgasgemischen enthaltene Methan teilweise aus dem oder den Produktgemischen abgetrennt wird.

11. Verfahren nach einem der vorstehenden Ansprüche, bei dem das oder die Produktgemische vor der zumindest teilweisen Abkühlung in dem oder den Wärmetauschern vorgekühlt werden.

12. Verfahren nach Anspruch 11, bei dem die Vorkühlung des oder der Produktgemische um 20 bis 100 Kelvin durchgeführt wird.

13. Anlage (100) zur Gewinnung von Ethylen aus Methan, mit Mitteln, die dazu eingerichtet sind, ein oder mehrere Methan enthaltende Einsatzgasgemische vorzuwärmen und einem oder mehreren Reaktoren (1), die dafür eingerichtet sind, einen Teil des in dem oder den Einsatzgasgemischen enthaltenen Methans durch oxidative Kopplung zu Ethylen umzusetzen, zuzuführen, wobei zum Vorwärmen des oder der Einsatzgemische ein oder mehrere Wärmetauscher (2) bereitgestellt sind, die dafür eingerichtet sind, ein oder mehrere Produktgemische, das oder die dem oder den Reaktoren (1) entnommen werden, abzukühlen, und zum Vorwärmen des oder der Einsatzgemische während eines Anfahrzeitraums zusätzlich ein mit Brennstoff befeuerter Gaserhitzers (3) bereitgestellt ist, **gekennzeichnet durch** Mittel, die dafür eingerichtet sind, das oder die Einsatzgasgemische während zumindest während des Anfahrzeitraums in paralleler Stromführung **durch** den oder die Wärmetauscher (2) und den Gaserhitzer (3) zu führen.

14. Anlage (100) nach Anspruch 13, die zur Durchführung eines Verfahrens nach einem der Ansprüche 1 bis 12 eingerichtet ist.
